Europäisches Patentamt

European Patent Office

Office européen des brevets

(19)

(11) Veröffentlichungsnummer : **0 353 218 B1**

(12) **EUROPÄISCHE PATENTSCHRIFT**

(45) Veröffentlichungstag der Patentschrift :
**27.05.92 Patentblatt 92/22**

(51) Int. Cl.$^5$ : **A61K 37/47, C12S 3/14**

(21) Anmeldenummer : **89890184.8**

(22) Anmeldetag : **10.07.89**

(54) **Verfahren zur Herstellung von Lys-Plasminogen.**

(30) Priorität : **28.07.88 AT 1919/88**

(43) Veröffentlichungstag der Anmeldung :
**31.01.90 Patentblatt 90/05**

(45) Bekanntmachung des Hinweises auf die
Patenterteilung :
**27.05.92 Patentblatt 92/22**

(84) Benannte Vertragsstaaten :
**AT BE CH DE ES FR GB IT LI LU NL SE**

(56) Entgegenhaltungen :
**HAEMOSTASIS, Band 18, Suppl. 1, 1988, Seiten 157-163, Basel, CH; A. SCHOPPMANN et al.: "Production and quality assurance of LYS-PLASMINOGEN steam treated"**

(56) Entgegenhaltungen :
**CHEMICAL ABSTRACTS, Band 106, Nr. 15, 13. April 1987, Seite 411, Zusammenfassung Nr. 117156d, Columbus, Ohio, US; Y. MAKINO et al.: "Conversion of Glu-plasminogen to Lys-plasminogen analysis by immunoblotting", & KETSUEKI TO MYAKKAN 1986, 17(4), 374-6 CHEMICAL ABSTRACTS, Band 105, Nr. 3, 21. Juli 1986, Seite 452, Zusammenfassung Nr. 222496b, Columbus, Ohio, US; J. YAMAMOTO et al.: "Production of Lys-plasminogen in urokinase-activated human plasma", & NIPPON KETSUEKI GAKKAI ZASSHI 1986, 49(1), 131-5**

(73) Patentinhaber : **IMMUNO Aktiengesellschaft
Industriestrasse 67
A-1221 Wien (AT)**

(72) Erfinder : **Linnau, Yendra, Dr.
Lavendelweg 24
A-1224 Wien (AT)**
Erfinder : **Hetzl, Ernst, Dr.
Bergheidengasse 8/1/9
A-1130 Wien (AT)**

(74) Vertreter : **Wolfram, Gustav, Dipl.-Ing.
Schwindgasse 7 P.O. Box 205
A-1041 Wien (AT)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Lys-Plasminogen, mit einer spezifischen Aktivität von mindestens 17,5 caseinolytischen Einheiten/mg Protein und mindestens 50 µmol/g Protein-Stickstoff sowie einer elektrophoretischen Reinheit von mindestens 90 %, wobei Plasminogen aus Plasma, einer plasminogenhältigen Fraktion oder einer Gewebekultur zwecks Reinigung an immobilisiertem Lysin adsorbiert, eluiert und aus dem Eluat mit einem Proteinfällungsmittel gewonnen wird.

Lys-Plasminogen ist ein in der Literatur verwendeter Sammelbegriff für proteolytisch modifizierte Formen des nativen Plasminogens (=Glu-Plasminogen), die aus diesem durch Abspaltung eines Polypeptides vom NH$_2$-Terminus erhalten werden. Als N-terminale Aminosäure der heute bekannten Species des Lys-Plasminogens wurden bisher Lysin, Methionin und Valin nachgewiesen. Als Molekulargewicht werden in der Literatur für Glu-Plasminogen Werte zwischen 90.000 und 94.000 und für Lys-Plasminogen Werte von etwa 80.000 angegeben.

Hinsichtlich des fibrinolytischen Geschehens unterscheidet sich Lys-Plasminogen von Glu-Plasminogen vor allem in zwei Punkten: Lys-Plasminogen besitzt eine höhere Bindungsaffinität an das Fibrinnetz von Thromben und kann um ein Vielfaches schneller zu Plasmin aktiviert werden (z.B. mit Urokinase). Beide Eigenschaften erhöhen die Effizienz der Fibrinolyse und sind der Grund dafür, daß Lys-Plasminogen bevorzugt in der Thrombose-Therapie verwendet wird.

Ausgangsmaterial für die Gewinnung von Lys-Plasminogen ist die Cohn-III-Fraktion des Plasmas oder Plasma selbst. Die in der Literatur beschriebenen Verfahren bestehen aus einer Vielzahl von Verfahrensschritten, die u.a. Extraktionen, Adsorptionen und Fällungen umfassen. Die Abspaltung einer N-terminalen Polypeptidkette und Umwandlung in Lys-Plasminogen wird entweder gezielt an isoliertem Glu-Plasminogen vorgenommen oder geschieht im Verlauf der Isolierungs- und Reinigungsschritte durch anwesende proteolytische Aktivität.

P.Wallen und B.Wiman (Biochim. Biophys. Acta 221, 20 - 30 (1970)) isolieren Plasminogen durch Extraktion eines gewaschenen Cohn III-Niederschlags und Fällung des Extraktes mit Methanol und Ammonsulfat bei pH 4,8. Dieses Produkt enthält 0,5 - 1,9 % spontane proteolytische Aktivität, die auf Verunreinigung mit Plasmin zurückzuführen ist. Nach Zusatz von Aprotinin liefert die weitere Reinigung durch Gelfiltration und Adsorption/Elution an einem Anionenaustauscher ein Plasminogen, das überwiegend Glutamin als N-terminale Aminosäure zeigt und eine spontane proteolytische Aktivität von 0,1 - 0,3 % aufweist. Eine Aufarbeitung ohne Zusatz von Aprotinin führt zu einem Produktgemisch mit einer spontanen proteolytischen Aktivität von 4 % und mit Lysin, Valin, aber auch Methionin und Glutamin als N-terminale Aminosäure.

E.E. Rickli und P.A. Cuendet (Biochim. Biophys. Acta 250, 447-451 (1971)) immobilisieren Lysin an Polyacrylamidgel und verwenden dieses Gel zur Isolierung von Plasminogen aus Plasma. Nach Waschen mit einem Phosphatpuffer erfolgt die Elution mit einem Puffer, der 0,5 mol/l 6-Aminocapronsäure enthält. Durch Zusatz von Ammonsulfat wird Plasminogen aus dem Eluat gefällt und der gelöste Niederschlag gegen NaCl-Tris-Lysin oder Phosphat-NaCl-Puffer dialysiert. Als N-terminale Aminosäure kann nur Glutamin nachgewiesen werden.

D.Collen und Mitarbeiter (Thromb.Res.7, 515 - 529 (1975)) beschreiben ebenfalls die Gewinnung von Plasminogen durch Affinitätschromatographie mit Lysin-Agarose aus Plasma bzw. Cohn III-Niederschlag mit und ohne Aprotininzusatz. Aus Plasma wird so mit Aprotinin reines Glu-Plasminogen gewonnen, ohne Aprotinin enthält das Produkt Spuren von Proteinen mit Lys und Val als N-terminale Aminosäure. Aus Cohn III-Niederschlag wurde ohne Aprotininzusatz ein Präparat mit einer spontanen proteolytischen Aktivität (hervorgerufen durch anwesendes Plasmin) von 0,1 - 1 % isoliert. Bei Zusatz von 25.000 - 50.000 KIE Aprotinin je kg Cohn III-Niederschlag und 5 KIE/ml zu den Wasch- und Elutionslösungen betrug die spontane proteolytische Aktivität unter 0,1 %, die Trennung von Glu- und Lys-Plasminogen erfolgte durch Ionenaustauschchromatographie an DEAE-Sephadex A-50.

H.Claeys und Mitarbeiter (Thromb.Res. 3, 515 - 523 (1973) und Biochim. Biophys. Acta, 342, 351 (1974)) extrahieren Plasminogen aus Cohn III-Niederschlag mit einem Phosphatpuffer (0,1 mol/l, pH 7,4), der 10 KIE Aprotinin/ml enthält. Nach Adsorption an Lysin-Agarose und Waschung mit einem Phosphatpuffer wird mit einem Puffer eluiert, der 6-Aminocapronsäure (0,2 mol/l) und Aprotinin 20 KIE/ml enthält. Das so isolierte Plasminogen hat eine spontane proteolytische Aktivität von etwa 0,4 % und als N-terminale Aminosäure neben Glu auch Lys und Spuren Val und Met. Bei der Aufarbeitung von Plasma nach der gleichen Methode wird als N-terminale Aminosäure nur Glu nachgewiesen, die spontane proteolytische Aktivität beträgt unter 0,1 %. Die weitere Reinigung erfolgt durch Gelfiltration (Sephadex G-150) und Ionenaustauschchromatographie (DEAE-Sephadex A-50). Bei Zusatz von Aprotinin (400 KIE/ml) vor der weiteren Reinigung wird auch mit Cohn III-Niederschlag als Ausgangsmaterial ein Endprodukt isoliert, das eine spontane proteolytische Aktivität von unter 0,1 % zeigt und Glu als N-terminale Aminosäure. Die Umwandlung von Glu-Plasminogen in Lys-Plasminogen gelingt den Autoren durch Inkubation mit Plasmin (Plasminogen/Plasmin 200 : 1 bis 20 : 3) bei 37°C und wird durch Zusatz von Trichloressigsäure oder Aprotinin gestoppt.

Alle diese Verfahren weisen Nachteile auf, weil sie sich entweder nicht zur Isolierung von Lys-Plasminogen in großem Maßstab eignen, da sie Verfahrensschritte, wie z.B. Gelfiltration umfassen, oder weil sie bei Raumtemperatur durchgeführt werden müssen, wodurch die Gefahr einer bakteriellen Kontamination des pharmazeutischen Präparates besteht. Meist führen die Verfahren auch zu Präparaten, die Plasmin und damit einen hohen Anteil an spontaner proteolytischer Aktivität enthalten. Genau das ist aber für eine therapeutische Verwendung unerwünscht, weil sie nach Applikation der Präparate auf Proenzyme des Gerinnungssystems einwirkt, sie teilweise zerstört und dadurch zu unerwünschten Nebenreaktionen führt. Diese proteolytische Aktivität kann zwar inhibiert werden, aber dann verunreinigen wiederum die entstandenen inaktiven Komplexe das hergestellte Präparat.

Die Erfindung bezweckt die Vermeidung dieser Nachteile und stellt sich die Aufgabe, Lys-Plasminogen mit einer spezifischen Aktivität von mindestens 17,5 caseinolytischen Einheiten pro mg Protein und mindestens 50 μmol Plasminogen pro g Stickstoff sowie einer elektrophoretischen Reinheit von mindestens 90 % herzustellen, wobei Plasminogen aus Plasma, einer plasminogenhältigen Fraktion oder einer Gewebekultur zwecks Reinigung an immobilisiertem Lysin adsorbiert, eluiert und aus dem Eluat mit einem Proteinfällungsmittel gewonnen wird.

Die erfindungsgemäße Aufgabe wird dadurch gelöst, daß eine Lösung des so gereinigten Plasminogens auf eine Plasminaktivität eingestellt wird, die gegenüber dem chromogenen Substrat H-D-Valyl-L-Leucyl-L-Lysin-p-nitroanilid-dihydrochlorid im Bereich von 0,005 bis 0,2 μmol/ml min, vorzugsweise 0,01 bis 0,1 μmol/ml min, liegt, bei einer Temperatur von +1°C bis +20°C, vorzugsweise +4°C bis +12°C, während einer Zeitdauer von 6 bis 60 Stunden, vorzugsweise 15 bis 50 Stunden, gehalten wird, um eine enzymatisch-proteolytische Umwandlung des Plasminogens in Lys-Plasminogen zu erreichen, worauf die Enzymeinwirkung unterbrochen und Lys-Plasminogen isoliert wird.

Zur erfindungsgemäßen Einstellung der Plasminaktivität können plasmatische Enzyme aus der Gruppe der Serinproteasen eingesetzt werden. Sie umfassen Plasmin und plasminähnliche Enzyme. Eine Plasminaktivität von 0,01 bis 0,1 μmol/ml min entspricht einem Plasminogen/Plasmin-Verhältnis von 30.000 : 1 bis 3.000 : 1. Daraus wird ersichtlich, daß die proteolytische Umwandlung des Glu-Plasminogens in Lys-Plasminogen mit überraschend kleinen Enzymmengen durchgeführt wird.

Vorzugsweise wird die Plasmineinwirkung auf das Plasminogen durch Zugabe von Inhibitoren unterbrochen.

Durch Verwendung äußerst geringer Mengen an plasmatischem Enzym reichen beim erfindungsgemäßen Verfahren auch geringe Mengen an Inhibitoren, um die Enzymeinwirkung zu unterbrechen. Durch den Zusatz von z.B. 10 bis 200 KIE/ml Aprotinin oder 0,01 bis 1,0 E/ml C1-Esterase-Inhibitor kann die Aktivität gegenüber dem Substrat S 2251 auf weniger oder gleich 0,03 μmol/ml min gesenkt werden. Dieser Wert ist ausreichend, um die Stabilität des Produktes während der weiteren Verarbeitungsschritte, wie Filtration, Gefriertrocknung und gegebenenfalls Hitzebehandlung zur Inaktivierung eventuell vorhandener Bakterien oder Viren, zu gewährleisten.

Zur Begrenzung der Plasmineinwirkung können auch andere Inhibitoren, wie alpha$_2$-Macroglobulin oder alpha$_2$-Antiplasmin verwendet werden.

Eine günstige Ausführungsform des erfindungsgemäßen Verfahrens besteht darin, daß die Plasmineinwirkung auf das Plasminogen während der Durchführung einer Dialyse vorgenommen wird.

Eine besonders günstige Variante des erfindungsgemäßen Verfahrens zur Herstellung von hitzestabilem Lys-Plasminogen aus Blutplasma oder Blutplasmaprodukten mit einer spezifischen Aktivität von mindestens 17,5 caseinolytischen Einheiten pro mg Protein, mindestens 50 nmol Plasminogen pro mg Stickstoff sowie einer elektrophoretischen Reinheit von mindestens 90 %, ist gekennzeichnet durch die Kombination der Maßnahmen, daß

a) eine Cohn Fraktion III (Niederschlag) mittels eines Phosphatpuffers in Anwesenheit von 0,1 bis 100 KIE Aprotinin pro ml Pufferlösung extrahiert wird, um eine Rohplasminogenfraktion zu erhalten,

b) die Rohplasminogenfraktion mit Äthanol behandelt wird, um den Hauptanteil der Nicht-Plasminogenproteine aus dem Extrakt zu fällen und zu entfernen,

c) aus der äthanolischen Lösung Plasminogen durch Adsorption an immobilisiertem Lysin und anschließender Elution isoliert wird,

d) das so gereinigte Plasminogen mittels Ammoniumsulfat oder Polyäthylenglykol (PEG) ausgefällt und zentrifugiert wird,

e) der Niederschlag gelöst, die Lösung in Gegenwart von Plasmin dialysiert wird, wobei die Lösung gegenüber dem chromogenen Substrat S 2251 eine Plasminaktivität von 0,01 bis 0,1 μmol/ml min aufweist, sodann

f) mit 10 bis 200 KIE Aprotinin die Enzymeinwirkung unterbrochen und

g) die Lösung danach lyophilisiert und das Lyophilisat gegebenenfalls hitzebehandelt wird.

Diese Ausführungsform geht somit von einer Cohn Fraktion III (Niederschlag) aus, wobei aus dieser Fraktion zuerst ein Extrakt - mit einem Phosphatpuffer - hergestellt wird. Dieser Extrakt enthält große Mengen an inerten Begleitproteinen, wie z.B. Immunglobuline und Lipoproteine, wobei vor allem letztere wegen schlechter Filtrationseigenschaften und unspezifischer Wechselwirkungen eine Reinigung mittels Affinitätschromatographie an immobilisiertem Lysin erschweren.

Durch eine Fällung mit Äthanol kann ein Großteil dieser störenden Begleitproteine entfernt werden.

Durch die Anwesenheit von 0,1 bis 100 KIE Aprotinin/ml Pufferlösung wird die Stabilität des Plasminogens während der affinitätschromatographischen Reinigung gewährleistet. Als Matrix für die Affinitätschromatographie wird ein Gel verwendet, das Lysin als Ligand enthält, vorzugsweise ein Lysin-Polyacrylamidgel. Nach der Adsorption werden die restlichen Begleitproteine durch Waschung - vorzugsweise mit einem Phosphatpuffer - entfernt und anschließend wird Plasminogen eluiert. Diese affinitätschromatographische Reinigung kann je nach Ansatzgröße in einer Säule oder in einem batch-Verfahren durchgeführt werden.

Durch Behandeln des Eluates mit Proteinfällungsmitteln, wie z.B. Ammonsulfat oder Polyäthylenglykol 4000, wird ein Zwischenprodukt ausgefällt, das in seinen Eigenschaften dem in der Literatur beschriebenen Glu-Plasminogen entspricht und in der SDS-PAGE ein Molekulargewicht von 92.000 zeigt.

Schon mit äußerst geringen Mengen an Plasmin kann dieses Zwischenprodukt in ein fibrinolytisches Proenzym übergeführt werden, das die in der Literatur beschriebenen Eigenschaften von Lys-Plasminogen aufweist und in der SDS-PAGE ein Molekulargewicht von 84.000 zeigt.

Durch wahlweise Zugabe von Plasmin oder Aprotinin kann die proteolytische Aktivität der Lösung des gereinigten Plasminogens (= Glu-Plasminogen) erhöht oder gesenkt werden, so daß der oben genannte Bereich von vorzugsweise 0,01 bis 0,1 µmol/ml min auf diese Weise sehr einfach eingestellt werden kann. Die Enzymwirkung in der Lösung wird nach der Umwandlung mit Aprotinin unterbrochen, die Lösung lyophilisiert und das Lyophilisat gegebenenfalls hitzebehandelt.

Die zur Durchführung des erfindungsgemäßen Verfahrens erforderlichen Methoden werden nachfolgend näher erläutert.

Bestimmung der spontanen proteolytischen Aktivität mit dem chromogenen Substrat H-D-Valyl-L-Leucyl-L-Lysin-p-nitroanilid-dihydrochlorid (S 2251, Firma Kabi)

Das Prinzip besteht darin, daß Plasmin und andere proteolytische Enzyme aus dem chromogenen Substrat p-Nitroanilin abspalten, dessen Freisetzung spektrophotometrisch als Zunahme der Extinktion bei 405 nm gemessen werden kann.

Die Proben werden zunächst im Verhältnis 1 : 2, 1 : 5, 1 : 10 und 1 : 20 vorverdünnt, wofür ein wässeriges Lösungsmittel mit pH = 7,2, enthaltend 10 mmol/l Lysin, 0,5 Gew.% PEG 6000 und 50 Gew.% Glycerin verwendet wird.

Das chromogene Substrat wird in destilliertem Wasser auf eine Konzentration von 5 mmol/l gelöst. Als Testpuffer dient eine wässerige Lösung vom pH = 7,2, enthaltend 50 mmol/l Tris und 180 mmol/l NaCl.

Zur Bestimmung werden 0,25 ml Probenverdünnung mit 0,55 ml Testpuffer gemischt, 2 min bei 37°C inkubiert, und nach Zusatz von 0,05 ml Substratlösung wird die Zunahme der Extinktion bei 405 nm bei einer Temperatur von 37°C verfolgt. Die Berechnung der Aktivität erfolgt nach der Formel

$$\Delta \, E/min.0{,}321.\text{Verdünnungsfaktor} \; = \; \mu mol/ml \; min$$

Bestimmung der spezifischen Aktivität von Plasminogen

a) Caseinolytische Einheiten (CU)/mg Protein

Dazu wird Plasminogen durch Umsetzung mit Streptokinase zu Plasmin aktiviert, das von Casein Fragmente abspaltet, die in Trichloressigsäure löslich sind und durch spektrophotometrische Messung der Extinktion im Überstand bei 280 nm erfaßbar sind.

Die Vorverdünnung der Proben auf eine Aktivität von 0,5 bis 2,5 CU/ml erfolgt mit einer wässerigen Lösung vom pH = 9,0, enthaltend 50 mmol/l Tris, 20 mmol/l Lysin, 0,1 mol/l NaCl und 1 mmol/l EDTA.

Das Substrat wird durch Lösen von Casein nach Hammarsten (4 %) in einem Phosphatpuffer (67 mmol/l, pH 7,4) hergestellt.

Zur Aktivierung wird eine Lösung von Streptokinase (2500 IE/ml) in Phosphatpuffer wie oben verwendet.

Zur Durchführung der Bestimmung werden im Eisbad 2,0 ml Caseinlösung, 1,4 ml Phosphatpuffer (67 mmol/l, pH 7,4), 0,4 ml Probenverdünnung und 0,2 ml Streptokinaselösung gemischt, wobei für den Blindwert statt der Probenverdünnung Puffer pipettiert wird. Nach 30-minütigem Erhitzen auf 37°C werden die Proben wieder ins Eisbad gestellt und das nicht gespaltene Casein wird durch Zusatz von 6 ml 15 % Trichloressigsäure ausgefällt. Nach mindestens 30-minütiger Stehzeit bei Raumtemperatur wird über Faltenfilter filtriert und die Extinktion im Überstand gegen den Blindwert bei 280 nm gemessen.

Die Berechnung der caseinolytischen Aktivität erfolgt nach der Formel

$$E_{280} \cdot 16,3 \cdot \text{Verd.} = \text{CU/ml.}$$

Es werden jeweils von mindestens zwei verschiedenen, innerhalb des Meßbereiches liegenden Verdünnungen, Doppelbestimmungen durchgeführt.

Die Umrechnung in die spezifische Aktivität (CU/mg Protein) erfolgt durch Division der caseinolytischen Aktivität durch den Proteinwert (Kjeldahl, in mg/ml).

b) Mikromol Plasminogen/g Protein-Stickstoff

Diese Bestimmungsmethode ist als "Active-Site-Cross-Titration" bekannt. Dazu wird zunächst Plasminogen durch Komplexbildung mit Streptokinase aktiviert. Dieser Komplex reagiert mit p-Nitrophenol-p′-guanidinobenzoat unter Freisetzung von p-Nitrophenol, das spektrophotometrisch durch Messung der Extinktion bei 400 nm bestimmt wird.

Folgende Reagenzien werden verwendet:

Testpuffer (pH = 7,7):      100 mmol/l $Na_2HPO_4$
                            1 mmol/l 6-Aminocapronsäure

Streptokinase-Puffer:       30 mmol/l $Na_2HPO_4$
                            150 mmol/l Na-Glutamat

Titrierlösung:              2,5 mmol/l p-Nitrophenyl-p′-guanidinobenzoat in trockenem DMSO (getrocknet über Molekularsieb $4.10^{-10}$m

Standardlösung:             1,0 mg p-Nitrophenol je 1 ml in trockenem DMSO. Vor der Verwendung werden 5 ml mit trockenem DMSO auf 100 ml verdünnt.

Zur Durchführung der Bestimmung werden 1 ml Testpuffer, 50 µl Streptokinaselösung (etwa 200 nmol/ml) und "V" µl Probe 20 bis 30 min bei 25°C inkubiert und die Extinktion bei 400 nm während 2,5 min gemessen. Nach Zusatz von 20 µl Titrierlösung wird gemischt und erneut die Extinktion bei 400 nm verfolgt. Die nach Zugabe der Titrierlösung gemessene Extinktionszunahme, sie soll zwischen 0,08 und 0,12 liegen, geht als "At" in die Berechnung ein.

Blanklauf: Statt Streptokinase wird Streptokinase-Puffer und statt der Probe wird das gleiche Volumen eines Puffers zugesetzt, der dieselbe Salzzusammensetzung wie die Probe hat, sonst erfolgt die Durchführung wie beim Testlauf. Die Extinktionszunahme geht als "Ab" in die Berechnung ein.

Standardlauf: Durchführung wie Blanklauf, statt der Titrierlösung wird aber Standardlösung zugesetzt. Die Extinktionszunahme geht als "As" in die Berechnung ein.

Die Berechnung der Plasminogenaktivität erfolgt mit der Formel

$$\frac{(At - Ab) \cdot 20 \cdot 0,00036 \cdot 10^6}{As \cdot V} = \mu\text{mol/l}$$

Durch Division dieser Aktivität mit dem Gehalt der Lösung an Protein-Stickstoff (Kjeldahl, in g/l) ergibt sich die spezifische Aktivität des Plasminogens in µmol/g Protein-Stickstoff.

Bestimmung des Molekulargewichtes (SDS-Polyacrylamid-Gelelektrophorese, SDS-PAGE)

Diese Methode besteht darin, daß durch Zusatz von SDS Proteine einheitlich geladen werden und in einem Polyacrylamidgel bei Anlegen einer elektrischen Spannung entsprechend ihrer Molekülgröße wandern. Durch Vergleich der Wanderungsweite mit der von Kalibratorproteinen mit bekanntem Molekulargewicht kann das Molekulargewicht der zu untersuchenden Proben bestimmt werden.

Zur Durchführung werden 47,5 g Acrylamid und 2,5 g N,N-Methylen-bis-acrylamid in einer wässerigen Pufferlösung vom pH-Wert 7,1, enthaltend 6 mol/l Harnstoff, 0,1 mol/l Tris und 0,1 % SDS(Natriumdodecylsulfat) gelöst.

57 ml dieser Lösung, 3 ml einer 0,5 % wässerigen Ammoniumperoxodisulfat-Lösung und 90 µl N,N,N′,N′-Tetramethyl-ethylendiamin werden rasch gemischt und in einen Gelgießstand gefüllt. Nach erfolgter Polymerisation wird das Gel in eine Elektrophoreseapparatur eingesetzt. Die Proben werden mit einer Lösung, die 9 mol/l Harnstoff und 4,5 % SDS enthält, auf eine Proteinkonzentration von 2,5 mg/ml verdünnt. Nach weiterer Verdünnung (1 + 1) mit Glycerin-Bromphenolblau werden je 6 µl Probe aufgetragen. Für die Elektrophorese wird ein Puffer vom pH = 7,1 verwendet, der 0,1 mol/l Tris, 0,1 % SDS und 0,001 % Natriumazid enthält. Die Elektrophorese wird während 10 min bei 50 V und anschließend 3 h bei 100 V durchgeführt. Anschließend wird das Gel während einer Stunde bei Raumtemperatur mit einer Lösung von 2 g Coomassie-Blau in 455 ml Methanol, 455 ml Wasser und 90 ml Eisessig gefärbt. Die Entfärbung erfolgt mit einer Mischung aus 2,5 l Methanol, 1,0 l Eisessig und 6,5 l Wasser.

Zur Bestimmung des Molekulargewichtes werden die Wanderungsweiten gemessen, diese sind proportional dem Logarithmus des Molekulargewichtes. Aus einem parallel analysierten Kalibrator wird so eine Eichgerade ermittelt, auf der das Molekulargewicht der zu untersuchenden Probe abgelesen werden kann.

Wenn in der zu untersuchenden Probe mehrere Proteinbanden zu erkennen sind, so wird deren Verteilung durch densitometrische Auswertung der Extinktion bei 601 nm bestimmt.

Herstellung von Lysin-Polyacrylamidgel

100 g Biogel P-300 (0,15 bis 0,30 mm, Polyacrylamidgel der Fa. Bio-Rad) werden in 2,6 l Wasser während 2 h bei 47°C gequollen und durch Umsetzung mit 1,2 l Hydrazinhydrat während 20 h bei 47°C in das Hydrazid übergeführt. Mit destilliertem Wasser wird bis zur Erreichung eines pH-Wertes unter 8,5 gewaschen (Filtration über Büchner-Trichter). Das gewaschene Gel wird in 6 l 0,3 N HCl suspendiert und der pH-Wert auf 1,1 korrigiert. Nach Kühlung auf 0°C wird eine kalte Lösung von 56 g Natriumnitrit in 200 ml Wasser zugesetzt und 3 min gerührt. Nach der Zugabe einer Lösung von 730 g Lysin in 2 l Wasser wird mindestens 3 h bei einer Temperatur von maximal +4°C und einem pH-Wert von 9,5 gerührt. Anschließend wird weitere 16 h bei +4°C gerührt. Nach dreimaligem Waschen mit je 20 l Wasser und Filtration über Büchner-Trichter wird mit 15 l einer Lösung, die 107 g/l Ammonchlorid enthält (pH = 8,8) 16 bis 20 h bei +4°C gerührt. Anschließend wird das Gel mit Wasser und danach noch mit einem Phosphatpuffer gewaschen.

Das erfindungsgemäße Verfahren wird anhand der folgenden Beispiele noch näher erläutert.

Beispiel 1:

1 kg Cohn III-Niederschlag wurde bei 0°C in 10 l eines Phosphatpuffers mit pH 7,4 suspendiert, dem vorher 10 KIE/ml Aprotinin zugesetzt worden waren. Unter Kühlung bis auf -2°C wurde Äthanol bis zu einer Endkonzentration von 10 % zugesetzt. Nach 15-stündigem Rühren bei einer Temperatur von -2°C wurde zentrifugiert und der Überstand über ein Tiefenfilter auf Cellulosebasis (AMF Cuno Zeta Plus 50 S) filtriert. Anschließend wurde mit 5 l Phosphatpuffer verdünnt und 500 g Lysin-Polyacrylamidgel eingeführt. Nach einer Rührzeit von einer Stunde bei 0°C wurde das mit Plasminogen beladene Gel durch Filtration über Büchnertrichter abgetrennt und so oft mit einem Phosphatpuffer gewaschen, bis im Filtrat kein Protein mehr nachweisbar war. Durch Rühren mit einer Lösung von 6-Amino-capronsäure (0,1 mol/l) in Phosphatpuffer wurde Plasminogen eluiert und anschließend durch Zusatz von 261 g Ammonsulfat je kg Eluat ausgefällt.

Der durch Zentrifugation gewonnene Niederschlag wurde in 32 ml eines isotonen Phosphat/Kochsalzpuffers gelöst. Da die Weiterverarbeitung zu der erfindungsgemäßen Präparation von der Gegenwart eines plasmatischen Enzyms, nämlich Plasmin, mit der angegebenen Aktivität abhängig ist, wird zunächst die Plasminaktivität der Lösung gegenüber dem chromogenen Substrat S 2251 gemessen. Sie betrug 0,045 µmol/ml, so daß in diesem Fall eine Korrektureinstellung nicht erforderlich war. In der SDS-PAGE konnte vor der Durchführung der anschließend vorgesehenen Dialyse nur eine Bande mit einem Molekulargewicht von 92.000 (= Glu-Plasminogen) nachgewiesen werden.

Nach der Dialyse gegen eine isotone Phosphat/Kochsalzlösung während 36 h bei einer Temperatur von 5,0°C wurden 50 KIE/ml Aprotinin zugesetzt. Die Aktivität gegenüber dem Substrat S 2251 betrug nun 0,01 µmol/ml min. Der Test auf caseinolytische Aktivität ergab 342 CU/ml. Dies entspricht bei einem Proteingehalt von 15,6 g/l (nach Kjeldahl) einer spezifischen Aktivität von 21,9 CU/mg. Die Active-Site-Cross-Titration lieferte eine Aktivität von 164 µmol/l und somit eine spezifische Aktivität von 65,7 µmol/g Protein-Stickstoff. Während der Dialyse konnte in der SDS-PAGE die Umwandlung des Proteins mit dem Molekulargewicht 92.000 (= Glu-Plasminogen) in ein Protein mit dem Molekulargewicht 84.000 (=Lys-Plasminogen) nachgewiesen werden. Der Anteil an diesem Produkt betrug 98 %.

Nach dem Zusatz von 20 mmol/l Lysin wurde ein pH-Wert von 7,0 eingestellt und danach filtriert und gefriergetrocknet. Der Wassergehalt des Lyophilisates wurde auf 7,8 Gew.% gebracht und anschließend zur Inaktivierung von eventuell vorhandenen Bakterien oder Viren 10 h auf 60°C erhitzt. Nach Lösen in destilliertem Wasser wurde sterilfiltriert, steril abgefüllt und erneut gefriergetrocknet. Das so hergestellte Endprodukt zeigte in den Tests auf spezifische Aktivität und elektrophoretische Reinheit gegenüber jenem Produkt, das unmittelbar nach der Dialyse erhalten wurde, unveränderte Ergebnisse.

Beispiel 2:

Die Extraktion von Cohn III-Niederschlag, Äthanolfällung und Filtration erfolgten wie in Beispiel 1. Nach der Verdünnung mit Phosphatpuffer wurde die Lösung durch eine Chromatographiesäule gepumpt, die mit 100 g Lysin-Polyacrylamidgel gepackt worden war. Waschen und Elution wurden mit den in Beispiel 1 beschriebenen Lösungen, aber in der Chromatographiesäule durchgeführt. Die Fällung aus dem Eluat mit Ammonsulfat und Lösen des Niederschlages erfolgte wie in Beispiel 1. In der SDS-PAGE wurde nur eine Bande mit Molekulargewicht von 92.000 nachgewiesen. Die Aktivität gegenüber dem chromogenen Substrat S 2251 betrug 0,003 μmol/ml min. Durch Zusatz von Plasmin wurde sie auf 0,016 μmol/ml min erhöht. Nach der Dialyse während 43 h bei 7°C und anschließendem Zusatz von 100 KIE/ml Aprotinin wurde in der SDS-PAGE ein Anteil der Bande mit einem Molekulargewicht von 84.000 (= Lys-Plasminogen) von größer 98 % gefunden. Die spezifische Aktivität betrug 22,3 CU/mg Protein bzw. 60,5 μmol/g Stickstoff.

Beispiel 3:

Die Extraktion von Cohn III-Niederschlag mit Phosphatpuffer wurde nach Zusatz von 2 KIE/ml Aprotinin durchgeführt. Die weitere Verarbeitung bis zur Fällung mit Ammonsulfat und Lösung des Niederschlages erfolgte wie im Beispiel 2.

Die Aktivität der Lösung gegenüber dem chromogenen Substrat S 2251 betrug 0,014 μmol/ml min. Nach der Dialyse während 22 h bei einer Temperatur von 11,7°C und anschließendem Zusatz von 20 KIE/ml Aprotinin wurde eine spezifische Aktivität von 20,5 CU/mg Protein bzw. 56,3 μmol/g Protein-Stickstoff gefunden, der Anteil an Lys-Plasminogen in der SDS-PAGE betrug 96 %.

Beispiel 4:

Die Isolierung von Plasminogen erfolgte bis einschließlich der Elution von Lysin-Polyacrylamidgel, wie in Beispiel 3 beschrieben. Durch Zusatz von 70 g Polyäthylenglykol 4000 zu 350 ml Eluat wurde Plasminogen ausgefällt. Der durch Zentrifugation gewonnene Niederschlag wurde in 15 ml eines Phosphat/NaCl-Puffers gelöst; in der SDS-PAGE konnte nur "Glu-Plasminogen" mit einem Molekulargewicht von 92.000 nachgewiesen werden. Nach Zusatz von Plasmin betrug die mit dem Substrat S 2251 gemessene Aktivität 0,01 μmol/ml min; es wurde 42 Stunden bei einer Temperatur von 6,6°C dialysiert, anschließend wurde Aprotinin (50 KIE/ml) zugesetzt.

Der Anteil an Lys-Plasminogen in der SDS-PAGE betrug 95 %; es wurde eine spezifische Aktivität von 23,4 CU/mg Protein bzw. 59,0 μmol/g Proteinstickstoff gefunden.

## Patentansprüche

1. Verfahren zur Herstellung von Lys-Plasminogen, mit einer spezifischen Aktivität von mindestens 17,5 caseinolytischen Einheiten/mg Protein und mindestens 50 mymol/g Protein-Stickstoff sowie einer elektrophoretischen Reinheit von mindestens 90 %, wobei Plasminogen aus Plasma, einer plasminogenhältigen Fraktion oder einer Gewebekultur zwecks Reinigung an immobilisiertem Lysin adsorbiert, eluiert und aus dem Eluat mit einem Proteinfällungsmittel gewonnen wird, dadurch gekennzeichnet, daß eine Lösung des so gereinigten Plasminogens auf eine Plasminaktivität eingestellt wird, die gegenüber dem chromogenen Substrat H-D-Valyl-L-Leucyl-L-Lysin-p-nitroaniliddihydrochlorid im Bereich von 0,005 bis 0,2 μmol/ml min, vorzugsweise 0,01 bis 0,1 μmol/ml min, liegt, bei einer Temperatur von +1°C bis +20°C, vorzugsweise +4°C bis +12°C, während einer Zeitdauer von 6 bis 60 Stunden, vorzugsweise 15 bis 50 Stunden, gehalten wird, um eine enzymatisch-proteolytische Umwandlung des Plasminogens in Lys-Plasminogen zu erreichen, worauf die Enzymeinwirkung unterbrochen und Lys-Plasminogen isoliert wird.

2. Verfahren nach Anspruch 1, dadurch gekennzeichnet, daß die Plasmineinwirkung auf das Plasminogen durch Zugabe von Inhibitoren unterbrochen wird.

3. Verfahren nach Anspruch 2, dadurch gekennzeichnet, daß als Inhibitoren zur Begrenzung der Plasmineinwirkung Aprotinin in einer Menge von 10 bis 200 KIE/ml, C1-Esterase-Inhibitor in einer Menge von 0,01 bis 1,0 E/ml, $alpha_2$-Macroglobulin oder $alpha_2$-Antiplasmin verwendet werden.

4. Verfahren nach Anspruch 1 oder 2, dadurch gekennzeichnet, daß die Plasmineinwirkung auf das Plasminogen während der Durchführung einer Dialyse vorgenommen wird.

5. Verfahren nach einem oder mehreren der Ansprüche 1 bis 4, zur Herstellung von hitzestabilem Lys-Plasminogen aus Blutplasma oder Blutplasmaprodukten mit einer spezifischen Aktivität von mindestens 17,5 caseinolytischen Einheiten pro mg Protein, mindestens 50 nmol Plasminogen pro mg Stickstoff sowie einer elektrophoretischen Reinheit von mindestens 90 %, gekennzeichnet durch die Kombination der Maßnahmen, daß

a) eine Cohn Fraktion III (Niederschlag) mittels eines Phosphatpuffers in Anwesenheit von 0,1 bis 100 KIE Aprotinin pro ml Pufferlösung extrahiert wird, um eine Rohplasminogenfraktion zu erhalten,

b) die Rohplasminogenfraktion mit Äthanol behandelt wird, um den Hauptanteil der Nicht-Plasminogenproteine aus dem Extrakt zu fällen und zu entfernen,

c) aus der äthanolischen Lösung Plasminogen durch Adsorption an immobilisiertem Lysin und anschließender Elution isoliert wird,

d) das so gereinigte Plasminogen mittels Ammoniumsulfat oder Polyäthylenglykol (PEG) ausgefällt und zentrifugiert wird,

e) der Niederschlag gelöst, die Lösung in Gegenwart von Plasmin dialysiert wird, wobei die Lösung gegenüber dem chromogenen Substrat H-D-Valyl-L-Leucyl-L-Lysin-p-nitroanilid-dihydrochlorid eine Plasminaktivität von 0,01 bis 0,1 µmol/ml min aufweist, sodann

f) mit 10 bis 200 KIE Aprotinin die Enzymeinwirkung unterbrochen und

g) die Lösung danach lyophilisiert und das Lyophilisat gegebenenfalls hitzebehandelt wird.

## Claims

1. Method of producing lys-plasminogen, having a specific activity of at least 17.5 caseinolytic units/mg protein and at least 50 µmoles/g protein nitrogen as well as an electrophoretic purity of at least 90 %, wherein plasminogen from plasma, a plasminogen-containing fraction or a tissue culture, is adsorbed on immobilised lysine for purification purposes, is eluted and recovered from the eluate with a protein precipitating agent, characterised in that a solution of the thus purified plasminogen is adjusted to a plasmin activity which, relative to the chromogenic substrate H-D-valyl-L-leucyl-L-lysine-p-nitroanilid dihydrochloride, ranges between 0.005 and 0.2 y mole/ml min, preferably between 0.01 and 0.1 µ mole/ml min, is maintained at a temperature of from +1°C to +20°C, preferably +4°C to +12°C, for a period of time of from 6 to 60 hours, preferably 15 to 50 hours, in order to achieve an enzymatic-proteolytic conversion of the plasminogen into lys-plasminogen, whereupon the enzymatic action is interrupted and the lys-plasminogen is isolated.

2. Method according to claim 1, characterised in that the action of plasmin on the plasminogen is interrupted by adding inhibitors.

3. Method according to claim 2, characterised in that, as inhibitors for limiting the action of plasmin, aprotinin in an amount of from 10 to 200 KIU/ml, Cl-esterase inhibitor in an amount of from 0.01 to 1.0 U/ml, alpha$_2$-macroglobulin or alpha$_2$-antiplasmin is used.

4. Method according to claim 1 or 2, characterised in that the action of plasmin on the plasminogen is realised while carrying out a dialysis.

5. Method according to one or more of claims 1 to 4, for producing heat-stable lys-plasminogen from blood plasma or blood plasma products, having a specific activity of at least 17.5 caseinolytic units per mg protein, at least 50 nmoles plasminogen per mg nitrogen as well as an electrophoretic purity of at least 90 %, characterised by the combination of the measures that

a) a Cohn fraction III (precipitate) is extracted by means of a phosphate buffer in the presence of from 0.1 to 100 KIU aprotinin per ml buffer solution, in order to obtain a crude plasminogen fraction,

b) the crude plasminogen fraction is treated with ethanol, in order to precipitate and remove the main portion of non-plasminogen proteins from the extract,

c) plasminogen is isolated from the ethanolic solution by adsorption on immobilised lysine and subsequent elution,

d) the thus purified plasminogen is precipitated by means of ammonium sulfate or polyethylene glycol (PEG) and is centrifuged,

e) the precipitate is dissolved, the solution is dialysed in the presence of plasmin, the solution having a plasmin activity of from 0.01 to 0.1 µ mole/ml min relative to the chromogenic substrate H-D-valyl-L-leucyl-L-lysine-p-nitroanilid dihydrochloride, then

f) the enzymatic action is interrupted with 10 to 200 KIU aprotinin and

g) the solution is, thereafter, lyophilised and, if desired, the lyophilisate is heat-treated.

## Revendications

1. Procédé de préparation de lys-plasminogène ayant une activité spécifique d'au moins 17,5 unités caséinolytiques/mg de protéine et d'au moins 50 µmol/g de protéine-azote ainsi qu'une pureté électrophorétique d'au moins 90%, du plasminogène émanant de plasma, d'une fraction contenant du plasminogène ou d'une culture tissulaire étant adsorbé en vue de sa purification sur de la lysine immobilisée, élué et extrait de l'éluat à l'aide d'un agent précipitant les protéines, caractérisé en ce qu'on ajuste l'activité plasmine d'une solution du plasminogène ainsi purifié vis-à-vis du substrat chromogène H-D-Valyl-L-Leucyl-L-Lysine-p-nitroanilide dichlorhydrate dans une plage de 0,005 à 0,2 µmol/ml min, de préférence 0,01 à 0,1 µmol/ml min, qu'on maintient à une température de +1°C à +20°C, de préférence de +4°C à +12°C pendant une durée de 6 à 60 h, de préférence de 15 à 50 h, pour obtenir une conversion enzymo-protéolytique du plasminogène en lys-plasminogène, après quoi on interrompt l'action de l'enzyme et on isole le lys-plasminogène.

2. Procédé selon la revendication 1, caractérisé en ce que l'action de la plasmine sur le plasminogène est interrompue par l'addition d'inhibiteurs.

3. Procédé selon la revendication 2, caractérisé en ce qu'on utilise comme inhibiteurs pour limiter l'action de la plasmine de l'aprotinine en une quantité de 10 à 200 KUI/ml, l'inhibiteur de la C1-estérase en une quantité de 0,01 à 1,0 U/ml, l'$\alpha_2$-macroglobuline ou l'$\alpha_2$-antiplasmine.

4. Procédé selon la revendication 1 ou 2, caractérisé en ce qu'on fait agir la plasmine sur le plasminogène pendant la réalisation d'une dialyse.

5. Procédé selon une ou plusieurs des revendications 1 à 4, de préparation à partir de plasma sanguin ou de produits de plasma sanguin de lys-plasminogène thermostable ayant une activité spécifique d'au moins 17,5 unités caséinolytiques par mg de protéine, au moins 50 nmol de plasminogène/mg d'azote ainsi qu'une pureté électrophorétique d'au moins 90%, caractérisé par la combinaison des mesures consistant à :

a) extraire une fraction de Cohn III (précipité) au moyen d'un tampon phosphate en présence de 0,1 à 100 KUI d'aprotinine/ml de solution tampon, pour obtenir une fraction de plasminogène brut,

b) traiter la fraction de plasminogène brut par l'éthanol pour précipiter et séparer de l'extrait la majeure partie des protéines quine sont pas du plasminogène,

c) isoler le plasminogène de la solution éthanolique par adsorption sur de la lysine immobilisée et élution subséquente,

d) précipiter le plasminogène ainsi purifié au moyen de sulfate d'ammonium ou de polyéthylèneglycol (PEG) et centrifuger,

e) dissoudre le précipité, dialyser la solution en présence de plasmine, la solution présentant vis-à-vis du substrat chromogène H-D-Valyl-L-Leucyl-L-Lysine-p-nitroanilide dichlorhydrate une activité plasmine de 0,01 à 0,1 µmol/ml min, puis à

f) interrompre l'action de l'enzyme à l'aide de 10 à 200 KUI d'aprotinine et

g) lyophiliser ensuite la solution et soumettre le lyophilisat le cas échéant à un traitement thermique.